# EUROPEAN PATENT APPLICATION

(11) **EP 1 535 882 A1**
(43) Date of publication of application: **01.06.2005**
(21) Application number: 04027412.8
(22) Date of filing: 18.11.2004
(51) Int. Cl.: C02F 1/467

(54) **Method for forwarding ozonated water**

(30) Priority: 26.11.2003 JP 2003395662
(71) Applicant: Siltronic AG, 81737 München (DE)
(72) Inventor: Haibara, Teruo, Yamaguchi, 743-0011 (JP); Uemura, Kenichi, Yamaguchi, 743-0075 (JP); Adachi, Takio, Misato-shi Saitama 341-0018 (JP); Tayadate, Toshio, Tokyo 115-0044 (JP)
(74) Representative: Rimböck, Karl-Heinz, Dr.

(57) **Abstract**

A method for forwarding ozonated water from an ozonated water producing device (6) to a use point (10) separated from the device and enabling the use point to be supplied with ozonated water having a concentration adjusted as aimed at, characterized by adding a diluting water (7) to a raw material ozonated water in the neighborhood of the ozonated water producing device (6) under a condition inhibiting exposure to an ambient air, adjusting the amount of the diluting water (7) to be added thereby giving the ozonated water (6) a target concentration at the use point (10), and subsequently guiding the ozonated water through a pipe (3) to the use point (10).

## Description

This invention relates to a method for forwarding ozonated water which is directed, where a use point exist at places separated from an ozonated water producing device, toward suppressing the decrease of the concentration of the ozonated water due to the self-decomposition of dissolved ozone and minimizing the loss of concentration.
Particularly, it relates to a method for forwarding ozonated water which is capable of minimizing the loss of ozone and allowing efficient supply of the ozonated water even when the self-decomposition of dissolved ozone by the ultra pure water of high purity is serious.

Ozone possesses a strong oxidizing power. Owing to this characteristic, it is utilized for sterilizing city water and swimming pools, sterilizing foodstuffs, bleaching pulp, and disposing of waste water. The use of ozone is continuing to increase in various fields. Since the techniques were developed for the production of ozone gas and ozonated water of high degree of cleanliness, their utilization has been rapidly expanding in the field of treating and cleaning silicon substrates and other semiconductor materials and liquid crystal panels.

The ozone gas is produced by a method for exposing oxygen gas to silent discharge and a method for electrolyzing ultra pure water, for example. The ozonated water is produced by dissolving in water the ozone gas produced by the method described above. As means to dissolve ozone in water, a method for bubbling ozone gas in water, a method for mixing ozone gas with water by means of an ejector, a method for dissolving ozone gas in water through the medium of a membrane made of Teflon (registered trademark for polyethylene fluoride type fiber), and a method for advancing ozone gas into counterflow contact with water flowing down the interior of a packed column have been known.

The ozone in the ozonated water transforms by self-decomposition into oxygen or dissipates as ozone gas into the atmosphere. When the ozonated water is left standing, it suffers loss of dissolved ozone concentration and eventually reverts to the original water. It is, therefore, customary to produce ozonated water in a high concentration and thereafter adjust the concentration of the ozonated water prior to use.

As a means to adjust the concentration of the ozonated water, a method for adjusting the amount of the ozone gas generated in an ozonated water producing device or adjusting the flow rate of the ozonated water during the course of production is available. The method which adjusts the concentration by adjusting the output of the ozonated water producing device is widely adopted because it permits most expeditious and accurate adjustment of the concentration. Since it attains the adjustment by reducing the capacity of the ozonated water producing device, it encounters difficulty in making the most of the capacity of the ozonated water producing device. It possibly incurs difficulty in producing a sudden change of concentration because it offers retarded response to the leading edge and the trailing edge of ozone concentration. A method which lowers the concentration to a level aimed at by promoting the decomposition of the ozonated water is also available for the adjustment of concentration. This method, however, has poor efficiency from the viewpoint of the effective utilization of the ozonated water because it incurs a very large loss of ozone. A method which relies on the dilution of the ozonated water is also available. Since this method has a very simple operating principle, it is actually used for dilution at the use point.

In recent years, as the ultra pure water used for cleaning semiconductor materials is steadily gaining in purity, it has become difficult to produce ozonated water of high concentration. Various grades of ultra pure water are shown in Table 1. The ultra pure water corresponding to the grade 64M is deprived of organic substance by decomposition induced by irradiation of ultraviolet light for the purpose of accomplishing the standard value of total organic carbon (referred to as TOC hereinafter). This irradiation of the ultraviolet light is thought to form one cause for rendering production of the ozonated water of high concentration difficult.

**Table 1:**

| Degree of accumulation and water quality required | | | | | | | |
|---|---|---|---|---|---|---|---|
| | | 64K | 256K | 1M | 4M | 16M | 64M |
| Specific resistance (MΩ·cm) | | >17 | >17.5 | >17.6-18 | >18.0 | >18.1 | >18.2 |
| Fine particles (pieces/ml) | >0.2 µm | <100 | <50 | | | | |
| | >0.1 µm | | | <10-20 | <5 | | |
| | >0.05 µm | | | | | <5 | <1 |
| | >0.03 µm | | | | | | <10 |
| Viable cells (cfu/l) | | <10³ | <10² | <10 | <5 | <1 | <1 |
| TOC (µgC/l) | | | <100 | <30-50 | <10-20 | <5 | <1 |
| Silica (µgSiO₂/l) | | | <10 | <5 | <3 | <3 | <1 |
| Dissolved oxygen (ugO/l) | | | <100 | <100 | <50 | <10 | <5 |

In the circumstances, methods for preparing the ozonated water of high concentration have been developed. A method which removes hydrogen peroxide, i.e. an ozone decomposing substance, to below 15 µg/L (Patent Document 1) and a method which removes an ozone decomposing substance with a redox catalyst (Patent Document 2 and Patent Document 3) are examples. As a means to prepare the ozonated water of high concentration by lowering the ratio of electrification of ultra pure water, a method of adding such an antistatic substance as an acidic substance or carbon dioxide to ultra pure water (Patent Document 4) is known. Further, as a means to prepare the ozonated water of high concentration by increasing the TOC concentration, a method which adds such a TOC component as isopropyl alcohol (IPA) or acetic acid till a concentration exceeding 15 ppb and not exceeding 250 ppb (Patent Document 5) has been known.

Incidentally, as a means to suppress the fall of the ozone concentration of the ozonated water during the supply of the ozonated water over a long distance, a method which forwards an ozone-dissolved water and undissolved ozone gas as a gas-liquid mixed fluid and separates this fluid into a gas and a liquid at the use point (Patent Document 6) has been known. This principle purports that even when the ozone on the liquid side self-decomposes during the course of the supply, the fall of the concentration of ozone is suppressed because the ozone on the gas side is dissolved again.
Patent Document 1: Official gazette of JP-A-2000-15272
Patent Document 2: Official gazette of JP-A-2000-308815
Patent Document 3: Official gazette of JP-A-2001-44162
Patent Document 4: Official gazette of JP-A-2000-262874
Patent Document 5: Official gazette of JP-A-2002-85947
Patent Document 6: Official gazette of JP-A-2001-291694

In Documents 1 - 3 concerning methods for removing ozone decomposing substances, while it is known that ozone is violently decomposed in a solution containing hydrogen peroxide, it is unclear how much the hydrogen peroxide supposed to be generated in a trace quantity by irradiation of the ultraviolet light promotes the decomposition of ozone. Further, the metallic pollution due to the passage through the redox catalyst of a metallic system of palladium, for example poses a problem.

Document 4 has a sole mention of "a method for producing ozonated water by dissolving ozone gas in ultra pure water through the medium of hollow yarns" which seems to induce large generation of electrification. The present inventors have been ascertained by their study that even by such other methods for the production of ozonated water as the method using a packed column and the method resorting to bubbling which seem to be affected little by electrification, it is not easy to produce the ozonated water of high concentration relative to the water of high purity. It is, therefore, inferred that the acid and carbon dioxide which are added as antistatic substances contribute to suppressing the decomposition of ozone by an action other than prevention of electrification. The pollution caused by the antibiotic substance added to the raw material ultra pure water also poses a problem.

In Document 5, since the TOC component is added to the ultra pure water which has effected necessary removal elaborately by irradiation of the ultraviolet light, the pollution with the TOC component is inevitable. That is, it is proper to remove the impurities in the ultra pure water to the fullest possible extent because the cleanliness of the wafer surface after the cleaning process is largely affected by the water quality of the ultra pure water to be used. By the same token, it is proper to remove the impurities in the ozonated water to the fullest possible extent. The conventional techniques mentioned above, therefore, are improper because they entail unavoidable pollution due to the process and the decline of purity with the additives.

Moreover, the method of Document 6 entails such problems as complicating the apparatus by essentially necessitating such special devices as a gas-liquid mixing unit and a gas-liquid separating unit, requiring supply of excess ozone gas, and possibly lowering the ozone concentration during the gas-liquid separation.

This invention aims to provide, when ozone or ozonated water of high concentration is obtained by making the most of the adjustment of the output of an ozonated water producing device, a method for efficiently forwarding the ozonated water to the use point by diluting ozone without being decomposed or dissipated.

It particularly aims to provide a method for effectively forwarding to the use point the ozonated water prepared by using ultra pure water known to incur violent self-decomposition of dissolved ozone.

It further aims to provide, when the ozonated water is forwarded over a long distance, a method for suppressing the loss of the ozonated water as by decomposition to the smallest possible extent and effectively utilizing the ozonated water.

The present inventors continued a diligent study concerning the change in the ozone concentration in the ozonated water prepared by dissolving ozone gas in an ultra pure water of high purity corresponding to 64 M. They have consequently found that the ultra pure water of high purity incurs no difficulty in solving ozone gas and exhibits a catalytic action in converting ozone into oxygen and that when the ozonated water of high concentration is diluted under a specific condition, the ozonated water of the optimum concentration can be efficiently supplied at the use point with the wasteful decomposition and dissipation of ozone suppressed. This invention has been perfected as a result.

The problems mentioned above are solved by the following items (1) - (6).
(1) A method for forwarding ozonated water from an ozonated water producing device to a use point and enabling the use point to be supplied with ozonated water having a concentration aimed at, characterized by adding a diluting water to a raw material ozonated water in the neighborhood of the ozonated water producing device under a condition inhibiting exposure to an ambient air, adjusting the ozone concentration to a level aimed at the use point, and subsequently guiding the ozonated water through a pipe to the use point.
(2) A method for forwarding ozonated water from an ozonated water producing device to a use point separated therefrom and enabling the use point to be supplied with ozonated water having a concentration aimed at, characterized by adding a first diluting water to a raw material ozonated water in the neighborhood of the ozonated water producing device under a condition inhibiting exposure to an ambient air thereby adjusting the ozone concentration to a level rather higher than the concentration aimed at in the neighborhood of the use point, subsequently guiding the ozonated water through a pipe to the neighborhood of the use point, and further adding a second diluting water thereby adjusting the ozone concentration at the level aimed at the use point.
(3) A method set forth in the preceding item (1) or (2), wherein the method of adding the diluting water to the raw material ozonated water under a condition inhibiting exposure to an ambient air consists in a method of adding the diluting water to the raw material ozonated water being forwarded through a closed pipe.
(4) A method set forth in any of the preceding items (1) - (3), wherein the raw material ozonated water has resulted from dissolving ozone in ultra pure water and the diluting water is ultra pure water.
(5) A method set forth in any of the preceding items (1) - (4), wherein the raw material ozonated water and/or the diluting water contain an ozone decomposition inhibiting agent.
(6) A method set forth in the preceding item (5), wherein the ozone decomposition inhibiting agent is at least one member selected from the group consisting of carbon dioxide gas, acidic substances, and organic substances.

This invention permits efficient use of the ozonated water at the use point with the wasteful decomposition of ozone suppressed. Moreover, since the method resides in diluting the ozonated water of high concentration, the adjustment of the output of the ozonated water producing device can be utilized to the utmost. Further, since the ozonated water of high concentration is diluted, the time spent for the forwarding of the ozonated water can be shortened.

Primarily this invention, in forwarding an ozonated water from an ozonated water producing device to the use point and enabling the use point to be supplied with an ozonated water of a target concentration, concerns a method for forwarding the ozonated water, characterized by adding a diluting water to a raw material ozonated water in the neighborhood of the ozonated water producing device under a condition inhibiting exposure to an ambient air, adjusting the ozone concentration to a level aimed at the use point, and subsequently guiding the ozonated water through a pipe to the use point.

The present inventors, with a view to learning the effect of dilution of the raw material ozonated water, tried to examine the ozone concentration in a water tank when the ozonated water was supplied to a water tank having an inner volume of 40 L for 10 minutes. To be specific, the ozonated water having an ozone concentration of 28 ppm was delivered at a rate of 4 L/min. over a distance of 30 cm and the ozonated water in the water tank was tested for ozone concentration after the elapse of 10 minutes. For comparison, an ozonated water having an ozone concentration of 56 ppm and ultra pure water were severally delivered each at a rate of 2 L/min. over the same distance for the same duration and the water in the water bath was tested for ozone concentration. The results of these two tests were compared. The ozone concentration was 9 ppm in the former test and 5 ppm in the latter test. This fact clearly indicates that the dilution of the ozonated water of high concentration with the ultra pure water results in promoting the loss of ozone as in the form of decomposition or dissipation of ozone and allows an inference that the ultra pure water manifests a catalytic action of promoting the effect of decomposing or dissipating the ozone contained in the ozonated water of high concentration, though the mechanism of this catalytic action remains yet to be elucidated. On the basis of these results, the dilution of the ozonated water of high concentration does not prove favorable.

When the study on the dilution with the ultra pure water of high purity was further continued, however, it was found that the loss of the ozone was suppressed by performing the dilution in a closed pipe. During the delivery of the ozonated water over a long distance, a search for the point in the entire length of the water pipe at which the decrease of the concentration of ozone caused by the dilution was smallest revealed that the loss by the decomposition of ozone was smaller when the ozonated water having the highest possible ozone concentration immediately after the production of the ozonated water was diluted and then forwarded to the use point than when the ozonated water of high zone concentration was forwarded to the use point and then diluted. Thus, the results indicate that also in the case of forwarding the ozonated water to a use point which is separated from the ozonated water producing device, the decrease of the concentration of the ozonated water due to the self-decomposition of the dissolved ozone can be suppressed, the loss of the concentration can be minimized, and the ozonated water can be efficiently utilized. Now, this invention will be explained in detail below.

The ozonated water producing device is only required to be capable of producing ozonated water and the method for producing the ozonated water and the raw material to be used for the production do not need to be particularly restricted. This invention, however, is characterized by diluting the ozonated water of high concentration and then forwarding it to the use point and is useful where a device capable of producing the ozonated water of high concentration is used. In the meantime, since the inclusion of a step for diluting the ozonated water results in allying the loss due to the decomposition and dissipation of ozone, the ozonated water production device does not need to be restricted on account of the ozone concentration. Generally, the ozone concentration is in the approximate range of 5 - 200 ppm.

In view of the present state of affairs which adds to the difficulty encountered in the production of ozonated water of high concentration in accordance as the ultra pure water used in cleaning a semiconductor material gains in purity, this invention is effective when the ultra pure water is used as the raw material for the ozonated water. It is known that particularly in the ultra pure water of high purity, the self-decomposition of the dissolved ozone occurs violently. This invention is highly effective when it is applied to the ozonated water which results from dissolving ozone in the ultra pure water of high purity. It is, therefore, effective when it is applied to such processes as cleaning and surface treating silicon substrates and other semiconductor materials and liquid crystal panels which require the ozonated water of high cleanliness.

The term "use point" as used in this invention is only required to be a point at which the ozonated water resulting from dilution is put to use. The purpose of the use and the distance between the ozonated water production device and the use point are irrelevant. Since this invention excels in the effect of allaying the loss of the ozone contained in the ozonated water, however, it is capable of manifesting the effect thereof fully satisfactorily when the distance between the ozonated water production device and the use point are long. Generally, the distance between the ozonated water production device and the use point are in the range of 1 - 20 m.

When the central supply system for ozone is realized in the future, the entire floor or the entire building will be supplied with the water. In that case, it is conceivable that the distance between the ozonated water production device and the use point will exceed 100 m.

This invention contemplates adding the raw material ozonated water prepared by the ozonated water production device and diluting water together in the neighborhood of the device. The expression "neighborhood of the ozonated water production device" means a point which lies between the ozonated water production device and the use point and falls within 1/2, preferably 1/5, more preferably 1/10, and particularly preferably 1/40 of the distance from the ozonated water production device toward the use point. By effecting this dilution on the raw material ozonated water production device side than on the use point side, the loss of the ozone contained in the raw material ozonated water can be prevented more effectively. Further, by effecting this dilution in the initial stage of the long-distance supply and increasing the flow volume thenceforth, it is made possible to shorten the time spent till the use point and allay the amount of the dissolved ozone to be decomposed.

Since the ozonated water is decomposed at a rate which increases in accordance as the concentration of the ozonated water increases, this invention proves advantageous in respect of the ability thereof to suppress the rate of decomposition of ozone by lowering the ozone concentration during the course of supply.

The dilution of the raw material ozonated water is attained by adding the diluting water to the raw material ozonated water under a condition inhibiting exposure to the ambient air. As a means to make this addition, a method of causing the raw material ozonated water to mix with the diluting water in a closed pipe is available. This method is implemented by introducing the raw material ozonated water supplied from the ozonated water production device into the pipe without being exposed to the ambient air, introducing the diluting water into the pipe via a diluting water mixing port provided in the pipe, and allowing the raw material ozonated water and the diluting water to mix with each other. By precluding the exposure of the raw material ozonated water to the ambient air, the decomposition of the dissolved ozone can be suppressed even when the ozonated water is mixed with the diluting water.

The amount of the diluting water to be added may be decided by taking into due account the loss of the ozone during the supply of the ozonated water till the use point. When the loss of the ozone is totally absent till the use point, the ozonated water concentration after the dilution in the neighborhood of the ozonated water production device may be set at the optimum concentration during the use at the use point. When the occurrence of the loss of α% in the ozonated water is revealed during the course of supply, the ozonated water is only required to have an ozone concentration (100 + α) /100 times higher than the ozonated water concentration at the use point while allowing the loss mentioned above.

This invention does not need to limit the frequency of dilution to one but allows the dilution to be made in a plurality of times. When the ozonated water is forwarded to the use point which are separated from the ozonated water production device and this use point is supplied with the ozonated water of a target concentration, for example, the first diluting water is added to the raw material ozonated water in the neighborhood of the ozonated water production device under a condition inhibiting exposure to the ambient air to adjust the ozone concentration to a level higher than the target concentration in the neighborhood of the use point, then the ozonated water is guided through the pipe to the neighborhood of the use point, and the second diluting water is added to the ozonated water to adjust the ozone concentration to the target concentration at the use point. When the loss of ozone of α% is present during the supply of the ozonated water, for example, the ozonated water of a concentration (100 + α + β)/100 times higher than the concentration of the ozonated water having a concentration of β% such as, for example, the ozonated water at the use point, is prepared by using the first diluting water and subsequently in the neighborhood of the use point, the ozonated water is adjusted by the addition of the second diluting water to the concentration optimum for actual use. Thus, the adjustment is effected at two stages as described above. By effecting the dilution as divided into a plurality of portions as described above, the ozone concentration at the use point can be adjusted finely and conveniently as well. The expression "neighborhood of the ozonated water production device" means a point which lies between the ozonated water production device and the use point and falls within 1/2, preferably 1/5, more preferably 1/10, and particularly preferably 1/40 of the distance from the ozonated water production device toward the use point.

This invention conveys the ozonated water by using a pipe. The pipe does not need to be particularly discriminated by the kind of material used for the pipe and the diameter of the pipe. For the purpose of preventing the ozone from being decomposed or dissipated in consequence of the exposure to the ambient air, the pipe requires to have a diameter suitable for the amount of the water to be conveyed. The water pipe is preferably furnished with a mechanism which adjusts the feed volumes and the feed rates of the raw material ozonated water and the diluting water. If the flow volume increases during the course of the supply of the water, this increase will possibly result in intensifying the decomposition of the ozonated water on the inner wall of the pipe and the joints in the pipe.

Though this invention fully manifests the effect on "the ozonated water resulting from dissolving ozone in ultra pure water of high purity" which is prone to severe ozone decomposition, the raw material ozonated water or the diluting water or both may add an ozone decomposition inhibiting agent. As the ozone decomposition inhibiting agent usable for this purpose, carbon dioxide gas, acidic substances, and organic substances are available.

This invention has the effect thereof enhanced when the raw material ozonated water of high concentration is used as diluted. In principle, however, this effect is manifested without reference to the concentration of the raw material ozonated water. The invention does not need to restrict the range of concentration of the raw material ozonated water or the ratio of dilution.

The method of this invention for forwarding the ozonated water contributes to the reduction of the cost of production of the ozonated water because it suppresses the decomposition of ozone by a simple operation, realizes the supply of the ozonated water over a long distance, and allows effective utilization of the ozonated water by lowering the loss thereof by decomposition.

It is, therefore, applied advantageously to the plants for producing semiconductor substrates and liquid crystal panels by consuming ozonated water of adjusted concentration in large amounts, particularly to the cleaning operations. Further, for the sake of exalting the convenience of the use of the ozonated water, the realization of the central supply of the ozonated water has been gaining in importance. This realization necessitates the supply over a long distance. Even in this case, the method of this invention is capable of effectively forwarding the ozonated water.

The method of this invention for forwarding the ozonated water can be utilized not merely in the field of semiconductors but in all the fields which are in need of using the ozonated water. These fields embrace detergent grade ozonated water for sterilizing and disinfecting foodstuffs and furnishings for medical practices and wash basin grade ozonated water for sterilizing and disinfecting various articles in numerous fields, for example.

Now, this invention will be described below with reference to working examples.

### Example 1:

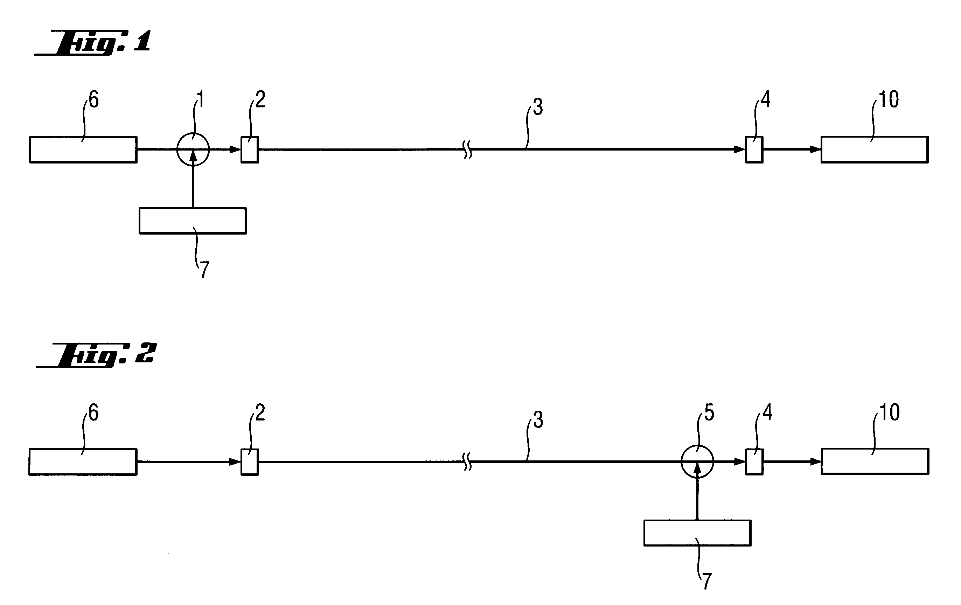

Ozonated water was prepared by using an apparatus illustrated in Fig. 1.

The raw material ozonated water produced by an ozonated water production device 6 was supplied through a Teflon (registered trademark) pipe 3, 1/2 inch in inner diameter to a use point 10. A diluting point 1 was disposed at a position of 0.5 m from the outlet port of the ozonated water production device 6. At this dilution point 1, the raw material ozonated water and the ultra pure water 7 for dilution were mixed inside the closed pipe without being exposed to the ambient air. A dissolved ozone concentration meter 2 was disposed at a position 0.5 m farther from the dilution point 1 and a dissolved ozone concentration meter 4 was disposed at a position 10 m farther from the dissolved ozone concentration meter 2. Between the dissolved ozone concentration meter 2 and the dissolved ozone concentration meter 4, a Teflon (registered trademark) pipe 3 of 1/2 inch in diameter was used for forwarding the ozonated water.

As the raw material ozonated water to be supplied, the solution of ozone in ultra pure water containing not more than 1 ppb of TOC was used. The flow rate of the raw material ozonated water was fixed at 2 L/min. Three flow rates, 2 L/min. (dilution to twice the original volume), 4 L/min. (dilution to three times the original volume), and 6 L/min. (dilution to four times the original volume) were used for the diluting ultra pure water. The raw material ozonated water and the diluting ultra pure water were both used at a fixed temperature of 25°C. The dissolved ozone concentrations were measured with the dissolved ozone concentration meter 2 (former stage ozone concentration) and the dissolved ozone concentration meter 4 (latter stage ozone concentration). The results are shown in Table 2.

### Comparative Example 1:

Ozonated water was prepared by using an apparatus illustrated in Fig. 2.

The raw material ozonated water produced by an ozonated water production device 6 was supplied through a Teflon (registered trademark) pipe 3, 1/2 inch in inside diameter to a use point 10. A dilution point 5 was disposed at a position 10.5 m from the outlet port of the ozonated water production device 6. At the dilution point 5, the raw material ozone water and the ultra pure water 7 for dilution were mixed in the closed pipe without being exposed to the ambient air. A dissolved ozone concentration meter 2 was disposed at a position of 1 m from the outlet port of the ozonated water production device 6 and a dissolved ozone concentration meter 4 was disposed 0.5 m farther from the dilution point 5 mentioned above.

The dissolved ozone concentrations were measured with the dissolved ozone concentration meter 2 and the dissolved ozone concentration meter 4 by following the procedure of Example 1 while mixing the raw material ozonated water at the dilution point 5 with the ultra pure water for dilution. The results are shown in Table 2.

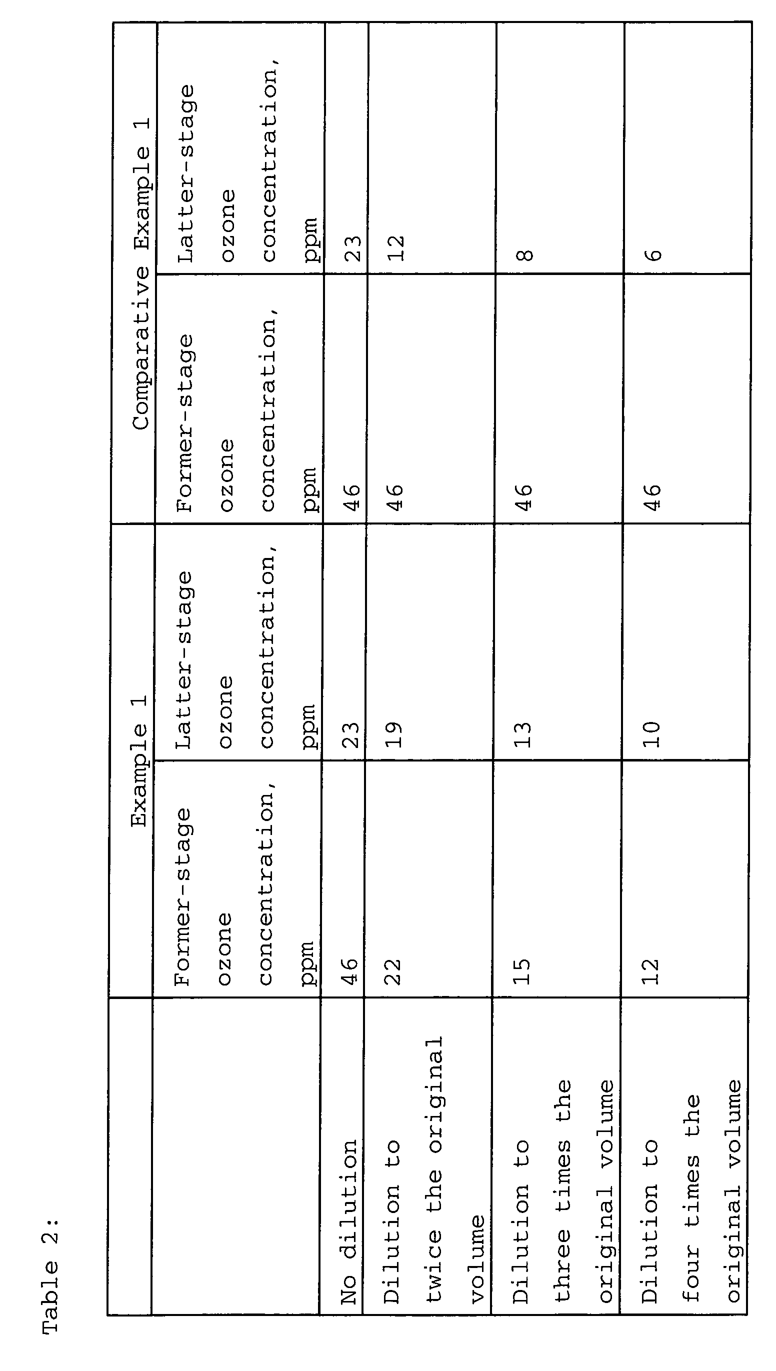

Comparison of Example 1 and Comparative Example 1 reveals that in the absence of dilution, while the concentrations of the raw material ozonated water (former-stage ozone concentrations) were 46 ppm, those at the use point (later-stage ozone concentrations) both fell to 23 ppm.

In contrast, in the dilutions to 2 - 4 times the original volume, the ozone concentrations at the use points varied with the positions of dilution. The ozone concentrations obtained in Example 1 in which the dilution was made in the neighborhood of the ozonated water production device were higher than the ozone concentrations obtained in Comparative Example 1 in which the dilution was made in the neighborhood of the use point.

### Example 2:

The supply of the ozonated water was carried out by following the procedure of Example 1 while using ozonated water prepared by dissolving carbon dioxide as an ozone decomposition inhibiting agent to pH 4.7 in ultra pure water having TOC lowered to 1 ppb by irradiation of the ultraviolet light. The results are shown in Table 3.

### Comparative Example 2:

The supply of the ozonated water was carried out by following the procedure of Comparative Example 1 while using ozonated water prepared by dissolving carbon dioxide as an ozone decomposition inhibiting agent to pH 4.7 in ultra pure water having TOC lowered to 1 ppb by irradiation of the ultraviolet light. The results are shown in Table 3.

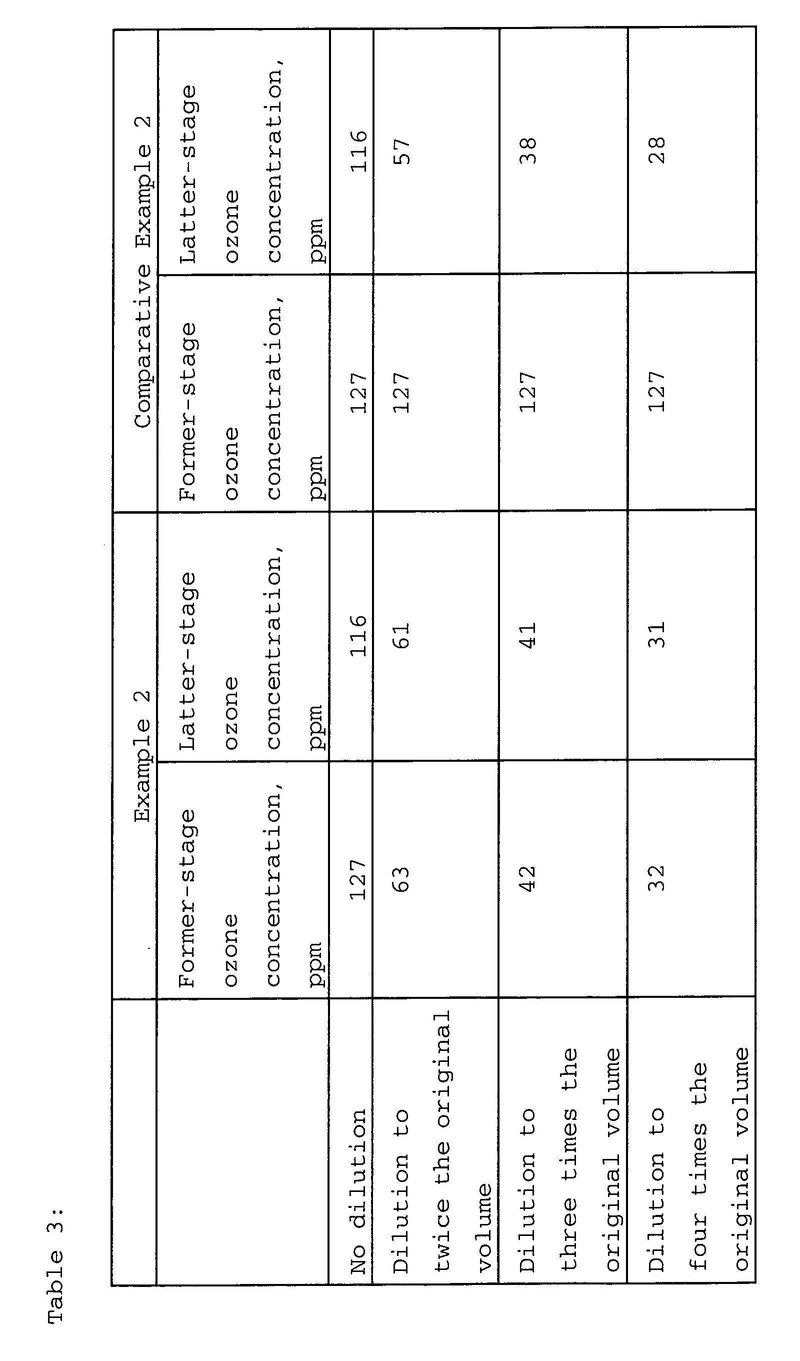

Comparison of Example 2 and Comparative Example 2 reveals that in the absence of dilution, while the concentrations of the raw material ozonated water (former-stage ozone concentrations) were 127 ppm, those at the use point (later-stage ozone concentrations) both fell to 116 ppm.

In contrast, in the dilutions to 2 - 4 times the original volume, the ozone concentrations at the use points varied with the positions of dilution. The ozone concentrations obtained in Example 2 in which the dilution was made in the neighborhood of the ozonated water production device were higher than the ozone concentrations obtained in Comparative Example 2 in which the dilution was made in the neighborhood of the use point.

### Example 3:

The supply of the ozonated water was carried out by following the procedure of Example 1 while using ozonated water prepared by dissolving isopropyl alcohol as an ozone decomposition inhibiting agent in a concentration of 1.5 ppm in ultra pure water having TOC lowered to 1 ppb by irradiation of the ultraviolet light. The results are shown in Table 4.

### Comparative Example 3:

The supply of the ozonated water was carried out by following the procedure of Comparative Example 1 while using ozonated water prepared by dissolving isopropyl alcohol as an ozone decomposition inhibiting agent in a concentration of 1.5 ppm in ultra pure water having TOC lowered to 1 ppb by irradiation of the ultraviolet light. The results are shown in Table 4.

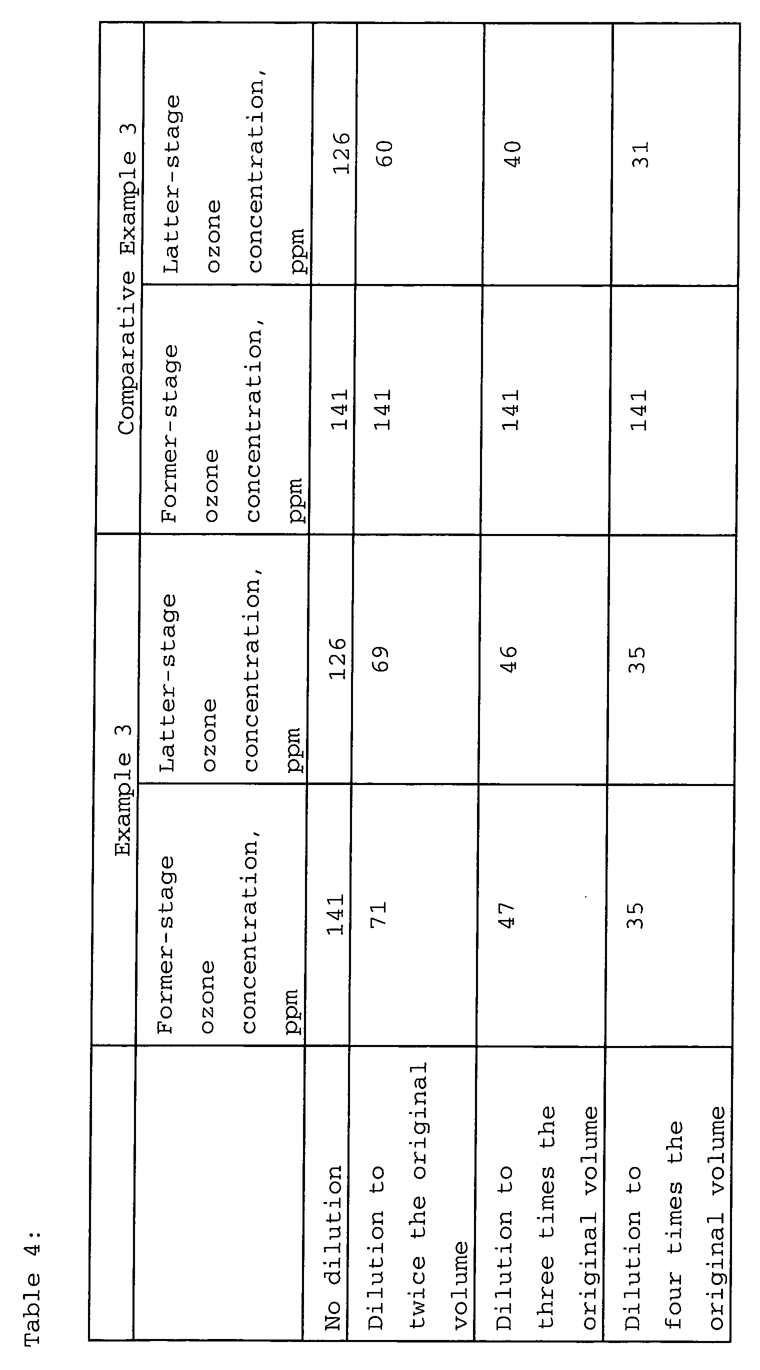

Comparison of Example 3 and Comparative Example 3 reveals that in the absence of dilution, while the concentrations of the raw material ozonated water (former-stage ozone concentrations) were 141 ppm, those at the use point (later-stage ozone concentrations) both fell to 126 ppm.

In contrast, in the dilutions to 2 - 4 times the original volume, the ozone concentrations at the use points varied with the positions of dilution. The ozone concentrations obtained in Example 3 in which the dilution was made in the neighborhood of the ozonated water production device were higher than the ozone concentrations obtained in Comparative Example 3 in which the dilution was made in the neighborhood of the use point.

According to the method of this invention for forwarding an ozonated water, in the case of forwarding the ozonated water over a long distance and enabling the use point to be supplied with the ozonated water having an adjusted concentration, a simple operation of adding the diluting water to the ozonated water immediately after production, guiding the resultant ozonated water through a pipe to the use point, and adjusting the amount of the diluting water to be added so as to enable the ozonated water to acquire a target concentration at the use point results in suppressing the decomposition of ozone, permitting the supply of the ozonated water over the long distance, lowering the loss of the ozonated water by decomposition, and allowing effective use of the ozonated water. Thus, the method of this invention contributes to the reduction of the cost of production of the ozonated water.

The method of this invention for forwarding the ozonated water, therefore, is applied advantageously to the plants for producing semiconductor substrates and liquid crystal panels by consuming ozonated water of adjusted concentration in large amounts, particularly to the cleaning operations.

Further, for the sake of exalting the convenience of the use of the ozonated water in the future, the realization of the central supply of the ozonated water has been gaining in importance. This realization necessitates the supply over a long distance. Even in this case, the method of this invention is capable of effectively forwarding the ozonated water.

The method of this invention for forwarding the ozonated water can be utilized not merely in the field of semiconductors mentioned above by way of an example but in all the fields which are in need of using the ozonated water. These fields embrace detergent grade ozonated water for sterilizing and disinfecting foodstuffs and furnishings for medical practices and wash basin grade ozonated water for sterilizing and disinfecting various articles in numerous fields, for example

### Brief description of the drawings:

Fig. 1: This is a diagram showing the positions for diluting the ozonated water and the positions for measuring the dissolved ozone concentrations in Examples 1 - 3.
Fig. 2: This is a diagram showing the positions for diluting the ozonated water and the positions for measuring the dissolved ozone concentrations in Comparative Examples 1 - 3.

### Explanation of reference numerals:

- 1:: Dilution point
- 2:: Dissolved ozone concentration meter
- 3:: Teflon (registered trademark) pipe 1/2 inch in diameter
- 4:: Ozone concentration meter
- 5:: Dilution point
- 6:: Ozonated water production device
- 7:: Ultra pure water
- 10:: Use point

## Claims

1. A method for forwarding ozonated water from an ozonated water producing device to a use point and enabling the use point to be supplied with ozonated water having a concentration aimed at, **characterized by** adding a diluting water to a raw material ozonated water in the neighborhood of the ozonated water producing device under a condition inhibiting exposure to an ambient air, adjusting the ozone concentration to a level aimed at the use point, and subsequently guiding the ozonated water through a pipe to the use point.

2. A method for forwarding ozonated water from an ozonated water producing device to a use point separated therefrom and enabling the use point to be supplied with ozonated water having a concentration aimed at, **characterized by** adding a first diluting water to a raw material ozonated water in the neighborhood of the ozonated water producing device under a condition inhibiting exposure to an ambient air thereby adjusting the ozone concentration to a level rather higher than the concentration aimed at in the neighborhood of the use point, subsequently guiding the ozonated water through a pipe to the neighborhood of the use point, and further adding a second diluting water thereby adjusting the ozone concentration at the level aimed at the use point.

3. A method according to claim 1 or claim 2, wherein the method of adding the diluting water to the raw material ozonated water under a condition inhibiting exposure to an ambient air comprising in a method of adding the diluting water to the raw material ozonated water being forwarded through a closed pipe.

4. A method according to any of claims 1 - 3, wherein the raw material ozonated water has resulted from dissolving ozone in ultra pure water and the diluting water is ultra pure water.

5. A method according to any of claims 1 - 4, wherein the raw material ozonated water and/or the diluting water contain an ozone decomposition inhibiting agent.

6. A method according to claim 5, wherein the ozone decomposition inhibiting agent is at least one member selected from the group consisting of carbon dioxide gas, acidic substances, and organic substances.
